# EUROPEAN PATENT APPLICATION

(11) **EP 3 248 636 A1**
(43) Date of publication of application: **29.11.2017**
(21) Application number: 16856457.3
(22) Date of filing: 26.05.2016
(51) Int. Cl.: A61M 5/31, A61M 5/32, A61M 39/24, A61M 5/165, A61M 5/34, B01D 35/02

(54) **FILTER ASSEMBLY FOR SYRINGE, AND SYRINGE AND RINGER ASSEMBLY COMPRISING SAME**

(30) Priority: 14.03.2016 KR 20160030376
(71) Applicant: Choi, Young Chul, Incheon 21971 (KR)
(72) Inventor: Choi, Young Chul, Incheon 21971 (KR)
(74) Representative: Arth, Hans-Lothar
(86) International application number: PCT/KR2016/005582
(87) International publication number: WO 2017/159923

(57) **Abstract**

Disclosed herein are a filter assembly for a syringe, a syringe including the same and a ringer assembly, which are able to filter and collect any impurity mixed with a medicinal fluid when the medicinal fluid is sucked from an ample into the syringe and allow to inject only the medicinal fluid while blocking the flow of the impurity when administrating the medicinal fluid in the syringe into a human body. The filter assembly for a syringe according to the present invention may include a needle holder configured to fix an injection needle at a front end thereof, wherein a neck part of a cylinder is inserted into a rear end thereof; and a filter unit which includes a support member which is formed in a single tube shape having a hollow part, wherein an outer side surface contacts close with the surrounding of the inner surface of the needle holder corresponding to the outer side surface is formed at a rear end in the longitudinal direction, wherein the support member is formed of a head part which forms one or more through holes passing through in the forward and backward directions and formed along the surrounding between the hollow part and the outer edge, and forms a hollow part communicating with the hollow part of the head part, and an extension tube which extends backward from the center of the back surface of the head part; a valve member which is formed in a horn shape, wherein a front end of the valve member forms a skirt part shaped to cover a circumferential portion of the through hole, and a rear end thereof which extends backward from the hollow circumferential portion of the back surface of the skirt part reaches the hollow rear end of the support member or is inserted in a protrusion shape and includes an extension part which forms a cutting line in the center of the rear end thereof; and a filter wherein a front opening formed in a pocket shape is engaged in a shape covering the side wall of the extension tube.

## Description

### TECHNICAL FIELD

The present invention relates to a filter assembly for a syringe, a syringe including the same, and a ringer assembly which are able to filter and collect any impurity mixed with a medicinal fluid when the medicinal fluid is sucked from an ample into the syringe and allow to inject only the medicinal fluid while blocking the flow of the impurity when administrating the medicinal fluid in the syringe into a human body.

### BACKGROUND ART

The syringe, in general, is configured to suck or discharge a medicinal fluid as a piston in a cylinder reciprocates. A needle holder for fixing an injection needle is detachably attached to a neck portion of the cylinder through which a medicinal fluid is sucked or discharged with respect to the cylinder.

Moreover, the aforementioned medicinal fluid is a mixture fluid of liquid and powder or a liquid and, in general, is stored in an ample made of a glass or plastic material. When in use, the neck portion of the ample is cut, and the ample is opened. Thereafter, an injection needle is inserted and sucks the medicinal fluid into the cylinder. If the mouth of the ample is sealed using a synthetic rubber, an injection needle is penetrated into the synthetic rubber and is inserted in the ample and sucks the medicinal fluid into the cylinder. In this way, the medicinal fluid is sucked.

The medicinal fluid sucked into the inside of the syringe may receive impurities, for example, the fragments of glass or plastic or the pieces of synthetic rubber, during the opening procedure of the ample. The impurities mixed in the syringe may be inputted into a human body together with the medicinal fluid.

The impurities injected into the inside of a human body may flow along the blood vessels of the human body and may hurt the inner walls of the blood vessels as well as the internal organs of the human body.

Intensive researches and efforts are underway to resolve the aforementioned problems. As an example of such researches and efforts, the Korean patent registration number 10-1245378 (registered on March 19, 2013, and this patent is referred to "a patent document 1") and the US patent registration number US4332249 (registered on June 1, 1982, this patent is referred to "a patent document 2").

The aforementioned patent documents 1 and 2 are referred to the structure of a check valve which allows the input of impurities during the procedure where a medicinal fluid is sucked into the syringe and filters such impurities in the discharge procedure of the medicinal fluid.

In the patent documents 1 and 2, the filter assembly is installed inside of the needle holder which is close to the injection needle, and the installation portion thereof corresponds to a portion where the inner diameter between the injection needle and an end of the cylinder neck is less than about 3.8∼4mm.

The aforementioned installation portion and the operational relationship will be described in detail with reference to the accompanying drawings.

As for the patent document 1 illustrated in Figures 1 and 2, a filter structure 10 is provided, wherein a suction port is formed at an outer side thereof, and a discharge port is formed in the center thereof. The suction port and the discharge port are configured in a check valve structure, both of which can be open in different directions.

The structure of the check valve of the discharge port is formed of a shielding curtain 12 which protrudes in a semispherical shape in the direction of the injection needle and has a cutting line in the center thereof, which turns into a hole as it is split wide.

Moreover, the shielding curtain 12 is made of a flexible material to provide the function of a check valve in response to the pressure which is applied from the inside of the cylinder.

More specifically, the shielding curtain 12 has a semispherical portion which corresponds to the injection needle "N" and is made of a flexible material and should be inevitably made in a very thin form in the center in the narrow space of less than 3.8∼4mm to provide the function of a check valve.

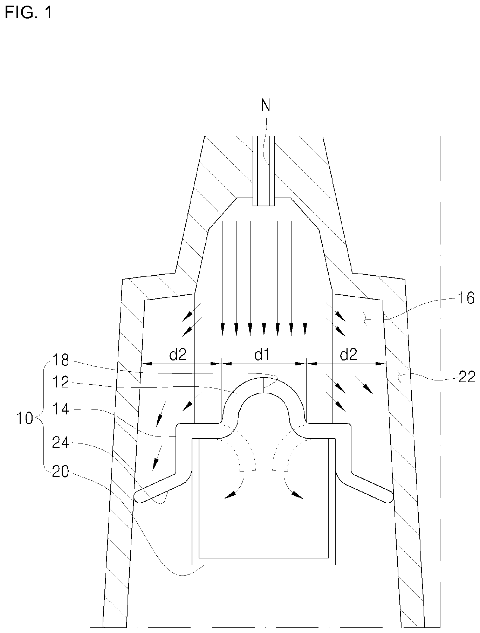

For this reason, the central region "d1" occupied by the shielding curtain 12, as illustrated in Figure 1, may directly receive the pressure of the medicinal fluid which is inputted via the injection needle "N".

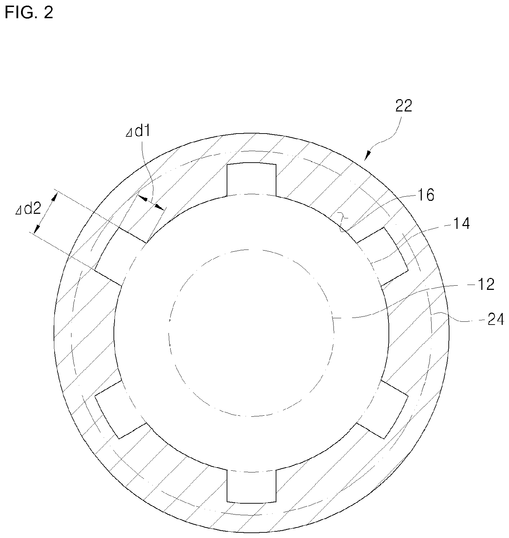

Furthermore, in the edge region "d2" which supports a cylindrical body 14 and defines a suction port, as illustrated in Figure 2, the space (Δd1 x Δd x n) between a plurality of pillar parts 16 should be formed narrow, and since it is covered with a rim curtain 24, the pressure of the medicinal fluid sucked via the injection needle "N" should be relatively lower as compared to the central region "d1" (d1>d2).

The pressure of the medicinal fluid flowing in the course of the procedure to suck the medicinal fluid into the cylinder is inevitably focused on the shielding curtain 12.

The shielding curtain 12 which receives the focusing of the pressure, is 0.1∼0.3 thick with respect to the cutting line 18 in the needle holder 22 of less than 3.8∼4mm. As illustrated by the dotted line in Figure 1, the semispherical shape of the shielding curtain 12 including the cutting line 18 may be transformed by the pressure or the cutting line 18 may be split wide. Impurities may be mixed together with the medicinal fluid and may gather between the shielding curtain 12 and the filter 20.

The impurities gathered between the shielding curtain 12 and the filter 20 may be mixed with the medicinal fluid during the administration of the medicinal fluid and may be discharged, for which the impurities may be inputted into the human body.

If the portion of the shielding curtain 12 is formed thicker so as to prevent the aforementioned problem, the cutting line 18 may be split wide when administrating the medicinal fluid.

According to the patent document 1, since the flow of the medicinal fluid is limited by the cutting line 18, a problem may occur, wherein the pressure exceeding a predetermined level is necessary using the piston, and the time for the reception and administration of the medicinal fluid unnecessarily increases.

Moreover, the patent document 1 describes that the filter assembly 10 may be tightly fit in the inner wall of the needle holder 22 or the pillar part 16; however the cylindrical body 14 which is tightly fitted, is made of a flexible material including the shielding curtain 12 and the rim curtain 24, which may cause a problem wherein the fixedly installed state may be removed if it receives the pressure of a medicinal fluid inputted into the inside of the cylinder during the suction procedure, and the space (Δd1 x Δd x n) defined between a plurality of the pillar parts 16 may become more narrowed.

In addition, the filter 20 of the patent document 1 is configured in an insertion type wherein it is inserted from the backside of the cylindrical body 14 to the inner side thereof. In this case, it is hard to retain a predetermined region in the cylindrical body 14 which is made of a flexible material including the filter 20 and to engage them.

Meanwhile, according to the patent document 2, a valve member 30 as illustrated in Figures 3 and 4 is formed of a suction part 34 which protrudes in the inner direction of the cylinder in the center and defines a slit 31 in the center thereof, and a skirt-shaped discharge part 36 formed at an outer portion thereof.

A filter 38 may be formed at the discharge part 36 at the inner side of the cylinder, wherein the filter 38 passes through a portion corresponding to the central portion of the valve member 30.

The suction part 34 and the discharge part 36 defined based on the central portion and the skirt are configured to open in the opposite direction to each other in response to the pressure which is applied to the inside of the cylinder.

In this configuration, if the medicinal fluid is sucked, the slit 32 of the central suction part 34 will be open, and the medicinal fluid is the cylinder and the impurities are mixed, and since the skirt (a discharge part 36) is contacting with the inner wall of the needle holder 40 in a state where it is supported by the filter 38, the contact of the impurities with the surface of the filter 38 can be prevented.

Moreover, in the course where the medicinal fluid in the cylinder is administrated to a human body, the slit 32 at the central portion is closed, and the impurities can be filtered by the filter 38 while it is discharged through a passage formed as the skirt portion with respect to the filter 38 is split wide.

According to the technical configuration of the patent document 2, it is basically referred to the configuration wherein the slit 32 is split open, and the medicinal fluid and the impurities are mixed in the course of the aforementioned suction procedure. In this case, the impurities may still remain stuck between the slits 32.

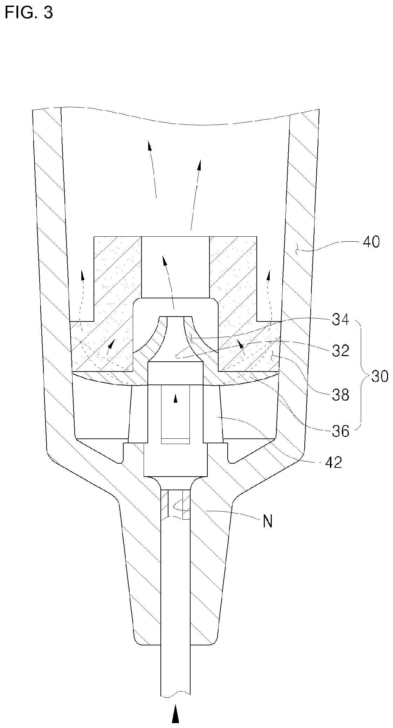

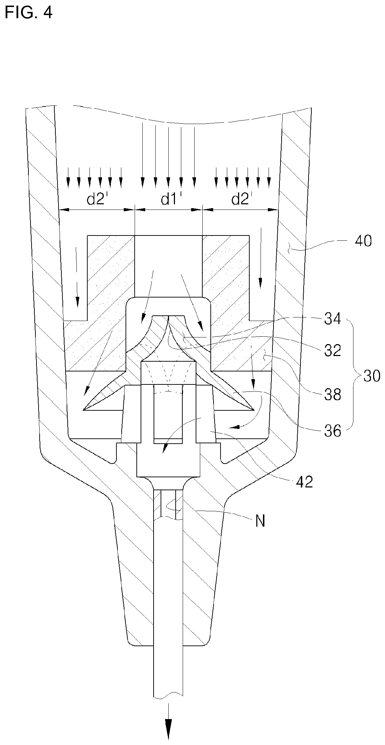

Furthermore, as illustrated in Figures 3 and 4, the installation position of the skirt (the discharge part 36) is determined based on a support part 42; however there may still be a problem since an outer end of the skirt (the discharge part 36) contacts with the inner wall surface of the needle holder 40 in the course of the suction procedure by means of the flexibility itself and the flexibility (since the flexibility of the material having a streamline property has an ordinary relationship) of the filter 38 which supports the backside thereof.

In case of the skirt (the discharge part 36), if the filter 38 is made of a flexible material which allows for a shape transformation, as indicated by the dotted line in Figure 3, impurities may be stuck during the suction between an outer end of the skirt (the discharge part 36) and the inner wall surface of the needle holder 40.

The impurities between the slits 32 or between the skirt and the inner wall surface of the needle holder 40 may be inputted into the human body together with the medicinal fluid in the course of the discharge.

Moreover, the filter 38 is configured in such a way that the filter 38 passes through the central region (d1') and carries out the filtering with respect to the outer side region of the central region (d1'), namely, the edge region (d2').

In the course of the discharge to administrate the medicinal fluid with the aid of the filter 38, the central region (d1') may receive relatively more pressure as compared to the edge region (d2') of the outer side thereof when considering the resistance of the filter 38 with respect to the flow of the medicinal fluid, which may cause a problem wherein the shape of the suction part 34 may be transformed in the opposite direction like in the patent document 1, while entailing another problem related thereto.

### DISCLOSURE OF THE INVENTION

Accordingly, the present invention is made in an effort to resolve the aforementioned problems. It is a first object of the present invention to provide a filter assembly for a syringe, a syringe including the same and a ringer assembly which are able to filter and collect impurities which are mixed when sucking a medicinal fluid into the inside of a cylinder of a syringe and prevent the collected impurities from entering a human body in the course where the medicinal fluid is administrated to the human body. It is a second object of the present invention which is able to enhance the reliability with respect to the filtering of impurities by stabilizing the installation structure. It is a third object of the present invention which allows for a reliable suction of the medicinal fluid into the inside of the cylinder of the syringe and a smooth flow of a medicinal fluid in the course of administration of the medicinal fluid with respect to a human body.

### Technical Solution

To achieve the above objects, there is provided a filter assembly for a syringe, which may include, but is not limited to, a needle holder configured to fix an injection needle at a front end thereof, wherein a neck part of a cylinder is inserted into a rear end thereof; and a filter unit which is disposed between the needle holder and the front end of the neck part, wherein the filter unit includes a support member which is formed in a single tube shape having a hollow part, wherein an outer side surface contacts close with the surrounding of the inner surface of the needle holder corresponding to the outer side surface is formed at a rear end in the longitudinal direction, wherein the support member is formed of a head part which forms one or more through holes passing through in the forward and backward directions and formed along the surrounding between the hollow part and the outer edge, and forms a hollow part communicating with the hollow part of the head part, and an extension tube which extends backward from the center of the back surface of the head part; a valve member which is formed in a horn shape, wherein a front end of the valve member forms a skirt part shaped to cover a circumferential portion of the through hole, and a rear end thereof which extends backward from the hollow circumferential portion of the back surface of the skirt part reaches the hollow rear end of the support member or is inserted in a protrusion shape and includes an extension part which forms a cutting line in the center of the rear end thereof; and a filter wherein a front opening formed in a pocket shape is engaged in a shape covering the side wall of the extension tube.

Moreover, first and second engaging protrusions are formed at the inner wall of the needle holder, while being correspondingly hung over the front surface edge and the back surface edge of the head part, and it is preferred that the skirt part of the valve member is configured in such a way that the outer edge is formed within an inner diameter formed by the first engaging protrusion.

Moreover, the needle holder is formed in a shape wherein the inner diameter thereof is gradually increasing in the backward direction, and the head part of the support member is formed in such a way that the front surface thereof is formed concave in the backward from the outer edge to the hollow part, and the extension tube of the support member is formed in such a way that the inner and outer diameters thereof are gradually decreasing in the backward direction.

In addition, a cover member may be further provided, wherein a rear end thereof forms a dome-shaped cover part covering an outer side of the rear end of the extension tube including the rear end of the extension part, wherein the cover member includes two or more than two cover lags which are disposed at an interval along the surrounding of the front end of the cover part and are formed extending and protruding in the forward direction, and the cover is configured to move toward the rear end in the filter in response to the flow of the medicinal fluid in the direction of the cylinder through the rear end of the valve member and is able to guide the flow of the medicinal fluid through the cover legs and moves forwardly in response to the flow of the medicinal fluid in the direction of the injection needle, and is able to guide the flow of the medicinal fluid in the directions of the through hole and the skirt part by covering the rear end of the extension tube.

To achieve the above objects, there is provided a syringe, which may include a cylinder which forms a single tube-shaped cylinder neck part at a front end thereof and a space to accommodate a medicinal fluid; a piston which is inserted through a rear opening of the cylinder and is able to move back and forth in the longitudinal direction, thus providing a flow pressure of the medicinal fluid to the inside of the cylinder based on the movement thereof; a needle holder of claim 1; and a filter unit of claim 1.

To achieve the above objects, there is provided a ringer assembly which may include a ringer body configured to accommodate a ringer's solution; a ringer pipe which forms a flow passage of the ringer's solution in the ringer body; a ringer connection tube which is engaged to the ringer pipe and is able to carry out the discharge of the ringer's solution; a needle holder of claim 1; and a filter unit of claim 1.

### ADVANTAGEOUS EFFECTS

According to the configuration of the present invention, the medicinal fluid and impurities which are mixed and inputted into the inside of the cylinder of the syringe are passed through the filter through an extension part of a valve member and a cutting line formed at the extension line, in which the separation and collection of the impurities are carried out by the filter. The impurities collected during the administration of the medicinal fluid to the human body can be isolated in the collected state, not affected by the flow of the medicinal fluid, thus increasing an injection prevention efficiency of impurities with respect to the human body can be enhanced.

Meanwhile, since the support member is equipped with a durability high enough to prevent any shape transformation in terms of its structure with respect to an installation pressure in response to the inner wall of the needle holder, an installation stability and a reliability of the filtering of impurities can be enhanced.

Moreover, in the course where the medicinal fluid is being sucked into the inside of the cylinder of the syringe, the flow of the medicinal fluid and the impurities can be oriented toward the filter while reducing any resistance, and the syringe can be easily used without applying over force in such a way to reduce the resistance with respect to the flow of the medicinal fluid in the course of the administration of the medicinal fluid, and it is possible to prevent the extension of time.

### BRIEF DESCRIPTION OF THE DRAWINGS

The present invention will become better understood with reference to the accompanying drawings which are given only by way of illustration and thus are not limitative of the present invention, wherein;
Figures 1 and 2 are cross sectional views for describing a technical configuration and an operational relationship of the patent document 1 corresponding to the conventional art;
Figures 3 and 4 are cross sectional views for describing a technical configuration and an operational relationship of the patent document 2 corresponding to the conventional art;
Figure 5 is a disassembled perspective view for describing a configuration of a filter assembly for a syringe and an engagement relationship thereof according to an embodiment of the present invention;
Figures 6A to 6C are cross sectional views for describing a configuration of a filter assembly for a syringe in Figure 5 and an engagement relationship of the configuration, and an operational relationship thereof;

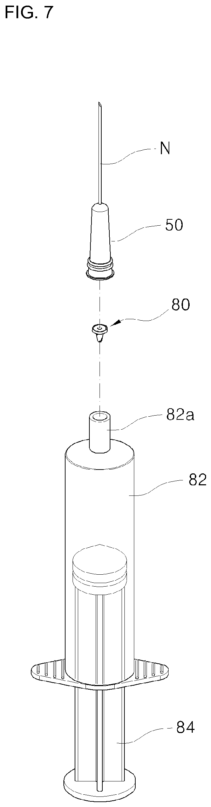

Figure 7 is a disassembled perspective view illustrating a configuration of a syringe equipped with a filter assembly in Figure 5; and

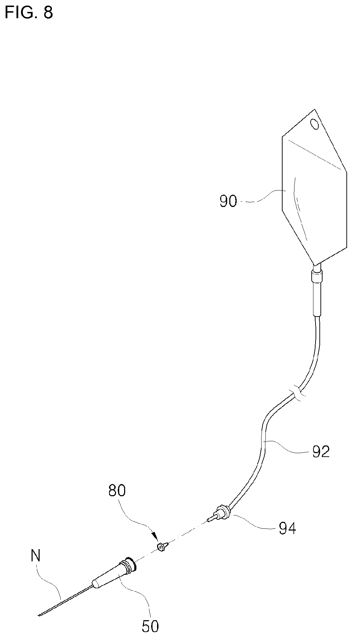

Figure 8 is a perspective view schematically illustrating a ringer assembly equipped with a filter assembly in Figure 5.

### MODES FOR CARRYING OUT THE INVENTION

In the descriptions of the present invention, the expression on the front side (a front end or a front surface) represents the direction where the injection needle is placed in the technical configuration in the completely assembled state or a portion where the direction is defined, and the backside represents the direction where the cylinder (a ringer connection tube) is placed at a front end of the injection needle or a portion where the direction is defined.

The preferred embodiments of the present invention will be described with reference to the accompanying drawings.

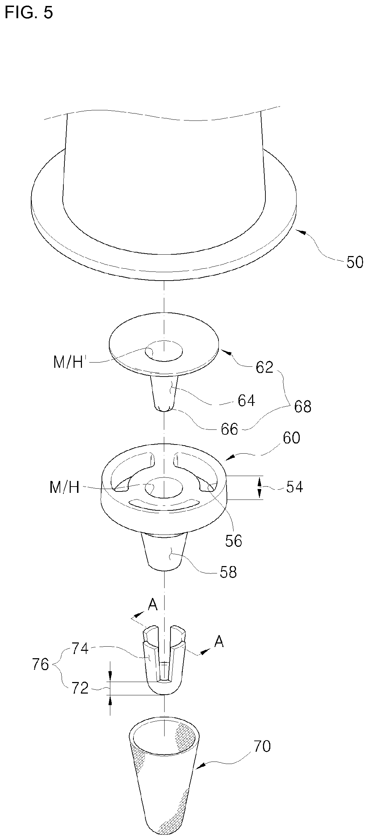

As illustrated in Figures 5 to 6C, the filter assembly for a syringe according to the present invention may include, but is not limited to, a needle holder 50 which is installed in such a way that the injection needle "N" is fixed at a front end thereof, and a neck part 82a of the cylinder 82 is inserted through the open rear end, and a filter unit 80 disposed between the needle holder 50 and the front end of the neck part 82a of the cylinder 82.

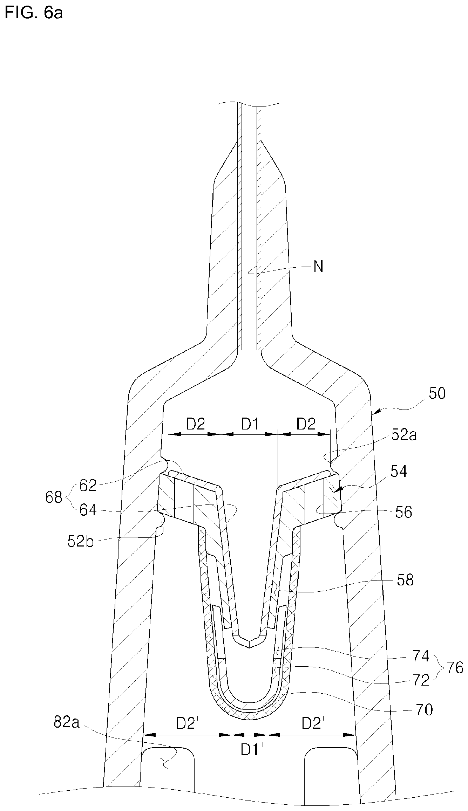

In the present invention, as illustrated in Figures 6A to 6C, the needle holder 50 includes a first engaging protrusion 52a or a shoulder (not illustrated) which formed protruding from the rear inner wall at a predetermined interval to where the injection needle "N" is fixed.

The first engaging protrusion 52a or the shoulder is employed to limit the forward installation position of the filter unit 80 including a support member 60 and to obtain an operation space of the skirt part 62 which forms a valve member 68. These components will be described in detail when describing the installation relationship between the needle holder 50 and the support member 60 and the operational relationship including the same, but these components are not essential components.

Moreover, a second engaging protrusion 52b may be formed at the inner wall of the needle holder 50 at an interval (which corresponds to the thickness of a head part 54 of the support member 60) from the first engaging protrusion 52a to the backside.

In addition, the needle holder 50 is preferably made of a synthetic resin material, and the inner diameter of the needle holder 50 may be configured in such a way that it is gradually expanded in the direction from the front position thereof to the backside, and an inclined angle corresponds to the tapered shape of the outer side of the neck part 82a of the cylinder of an ordinary syringe and is preferably determined within a range of a minus slope which makes easier the extraction during the injection and molding procedure of the needle holder 50.

The first engaging protrusion 52a or the shoulder and the second engaging protrusion 52b may be formed protruding in a circular shape along the rim from the previously set inner wall position of the needle holder 50.

Moreover, the first engaging protrusion 52a or the shoulder and the second engaging protrusion 52b may be formed in multiple numbers at a predetermined interval along the inner wall surrounding of the needle holder 50 in the shape of the pillar part 16 of the patent document 1 in the descriptions of the conventional art.

The support member 60 is not inserted into the front inner side which is formed by the first engaging protrusion 52a or the shoulder of the pillar part 16 of the patent document 1, but is hung over the end of the backside of the first engaging protrusion 52a or the shoulder and is hung over the front portion of the second engaging protrusion 52b.

The first engaging protrusion 52a and the second engaging protrusion 52b may be formed by a so-called undercut (which corresponds to an inwardly protruding shape) which allows for a forced extraction during the injection and molding of the needle holder 50.

Meanwhile, as illustrated in Figure 5, the filter unit 80 installed at the needle holder 50 may include, but is not limited to, a support member 60 equipped with a hollow part "M/H" in the center and a through hole 56 at an edge thereof and configured to receive an installation support in response to the needle holder 50, a valve member 68 configured in a horn shape (or a funnel shape) having a hollow part corresponding to the front portion of the support member 60 and the hollow part "M/H" of the support member 60 and equipped with a cutting line 66 at the rear end thereof, and a filter 70 which is installed in a shape covering the rear end of the support member 60 including the rear end of the valve member 68 in a pocket shape.

The support member 60 among the components forming the filter unit 80 may form a hollow part "M/H" passing in the forward and backward directions a center portion, and may include a head part 54 which has a thickness in the forward and backward directions at the front portion with respect to the longitudinal direction center of the hollow part "M/H" and is formed in a shape protruded extending outward, wherein the side surface of the outwardly protruding portion contacts close along the surrounding of the inner surface of the needle holder 50 at a previously set position.

Moreover, the support member 60 may form a tubular extension tube 58 which extends backward from the hollow circumferential surface of the head part 54.

At least one through hole 56 passing through in the forward and backward directions is formed between the hollow part "M/H" of the head part 54 and the surrounding of the edge of the outer side thereof.

When designing, it is preferred that the outer line of the through hole 56 with respect to the center of the hollow part "M/H" may position at a portion shorter than an interval where the first engaging protrusion 52a or the shoulder is crossing with respect to the center of the hollow part "M/H" at a previously set installation position, and the inner line of the through hole 56 may position at a portion being over the outer side of the extension tube 58 at a portion where the thickness necessary to maintain the shape of the hollow part "M/H" can be obtained.

It is preferred that the through hole 56 is provided in the number of two or more than two along the direction of the surrounding with respect to the hollow part "M/H", and the portion between the neighboring through holes 56 in the direction of the surrounding with respect to the hollow part "M/H" may be preferably formed in the shape of a spoke wherein the edge portion of the head part 54 is defined as a rim, and the circumferential portion of the hollow part "M/H" is defined as a boss.

As illustrated in Figures 5 to 6c, the head part 54 is preferably manufactured in such a way that at least front side between the front side and the backside of the head part 54 is formed gradually sinking in the backward direction. The reason for this configuration will be described in detail when describing the installation relationship and the operational relationship in relation with the present invention.

It is preferred that the side surface of the outer side of the head part 54 is formed in a tapered shape having the angle in the same or in the same range as the angle that the inner wall surface of the needle holder 50 forms so as to provide a close contact corresponding to the inner wall surface of the needle holder 50 at a previously set position.

It is preferred that the extension tube 58 is formed in a taper shape at the outer surface as well as the inner surface thereof wherein the thickness is gradually decreased in the direction of the backside thereof. It is enough to form in a shape corresponding to a so-called minus slope during the injection and molding procedure of the support member 60.

Meanwhile, the valve member 68 among the components forming the filter unit 80 is made of a silicon or a synthetic rubber material having flexibility, and is formed in a horn shape wherein the center thereof in whole has a hollow part "M/H"', and the front end formed based on the horn shape may form a skirt part 62 shaped covering the circumferential portion of at least the through hole 56 in the front surface of the support member 60, and the rear end extending backward along the circumferential portion of the hollow part "M/H"' at the rear surface of the skirt part 62 may form an extension part 64 which is inserted from the front side to the backside with respect to the hollow part "M/H" of the support part 60.

When designing, it is preferred that the position of the outer edge of the skirt part 62 is defined at a portion which is over the outer line of the through hole 56 at the inner side which does not contact with the first engaging protrusion 52a or the shoulder in a state where the valve member 68 is assembled with the support member 60.

Moreover, the rear end of the extension part 64 may position at the inner side of the extension tube 58 approaching the rear end of the extension tube 58 of the support member 60 when it is inserted into the hollow part "M/H" of the support member 60 or may be laid protruding backward from the rear end of the extension tube 58.

The rear end of the extension part 64 may be formed in a dome shape or a blocked shape like the end of a flat screw driver when viewing its exterior, and a cutting line 66 may be formed in the center of the rear end of the extension part 64, wherein the cutting line 66 can be split wide as it passes from the forward and backward directions, and pressure is applied from the front side to both sides which have been passed through.

Moreover, it is preferred that the skirt part 62 is formed in a predetermined shape corresponding to the shape wherein the front side of the support member 60 is formed sunk in the backward direction, and the front side and backside of the skirt part 62 are formed inclined toward the hollow part "M/H"' and the backside thereof.

The shape of the skirt part 62 can be easily transformed in the forward direction, but the transformation thereof is not easy with respect to the backside. More specifically, it is in a relationship supported in a close contact state of the support member 60.

The edge portion defined by the skirt part 62 is formed sunk in the directions of the hollow part "M/H" and the backside like the support member 60 so that the flow of the medicinal fluid inputted through the injection needle "N" can concentrate on the portion of the hollow part "M/H"' and at the same time the rear end of the extension part 64 can easily split open by the pressure thereof.

At this time, when designing, it is preferred that the inner diameter at the rear end of the extension tube 58, the inner diameter of the extension part 64 at the position thereof, and the cutting line 66 are split wide by the flowing pressure of the medicinal fluid in such a way that the widening thereof is over the inner diameter of the injection needle "N".

Meanwhile, the filter 70 among the components forming the filter unit 80 is made of a fiber material and may be made in the shape of a pocket having an opening in the forward direction.

The filter 70 is installed in such a way that in a state where the rear end of the support member 60 including the rear end of the valve member 68 is inserted into the inside of the filter 70 through the opening of the filter 70 based on the shape of the pocket, the open front end of the filter 70 covers the front side wall of the extension tube 58 approaching the head part 54 of the support member 60.

The filter 70 may be engaged by a melting method which uses the heat in response to the side wall of the front side of the extension tube 58 or a method of using a bonding, a hot melt, etc. It is preferred that the material used as an adhesive is harmless to a human body in terms of a chemical reaction with a medicinal fluid.

When designing, it is preferred that the thusly installed filter 70 can maintain over a previously set interval with respect to the rear end of the support member 60 including the rear end of the valve member 68 if the interval to the inner rear end from the opening, namely, the depth of the filter 70 is referred to the engaging position.

The interval from the rear end of the support member 60 including the rear end of the valve member 68 to the rear end at the inner side of the filter 70 may be determined in a range where the rear end of the extension part 64 of the valve member 68 can be at least split wide with respect to the cutting line 66.

The depth of the filter 70 may be determined satisfying a condition where the rear end of the extension part 64 of the valve member 68 can be split wide sufficiently with respect to the cutting line 66, thus securing a movable range of the cover member 76 which could be additionally provided.

Meanwhile, the filter unit 80 may further include, between the rear end of the support member 60 including the rear end of the valve member and the rear end at the inner side of the filter 70, a cover member 76 which is equipped with a dome-shaped cover part 72 to cover the rear end of the support member 60 including the rear end of the valve member 68 based on the forward and backward movement positions.

Moreover, the cover member 76 may include a dome-shaped cover part 72 a rear end of which is configured to cover the outer side of the rear end of the support member 60 including the rear end of the valve member 68, and a cover leg 74 which is provided by two or more than two at a predetermined interval along the surrounding of the front end of the cover part 72 and extends protruding forward.

While the medicinal fluid is being sucked in the direction of the cylinder 82, the cover member 76 moves in the direction of the rear end in the filter 70 by the flow pressure of the medicinal fluid inputted through the rear end of the valve member 68, and in case of the cover member 76, the front end of the cover part 72 will retract to the position exposed from the side portion of the rear end of the valve member 68, and the medicinal fluid inputted through the portion of the cutting line 66 of the rear end of the valve member 68 will flow toward the cylinder 82 through between the cover legs 74.

Meanwhile, during the administration procedure of the medicinal fluid, the cover member 76 may move forward by receiving the flow pressure of the medicinal fluid transferred through the neck part 82a of the cylinder 82, and the cover part 72 may cover the outer side of the rear end of the support member 60 including the rear end of the valve member 68 as the cover leg 74 receives the guide of the extension tube 58, by which the flow pressure of the medicinal fluid discharged from the inside of the cylinder 82 can be concentrated on the through hole 56.

The installation relationship and operation relationship with respect to the filter assembly for a syringe according to the present invention will be described.

First, the filter unit 80 may be installed in such a way that it is inserted with over a predetermined set level of pressure in the forward direction of the inside thereof through the rear opening of the needle holder 50 in a state the filter unit has been fixed at a jig.

At this time, the head part 54 of the support member 60 forming the filter unit 80 is manufactured in such a way that a portion of the side surface thereof contacts close to a previously set inclined inner surface of the needle holder 50, it may be installed through a tight fitting without forming the first engaging protrusion 52a or the shoulder.

In this case, the first engaging protrusion 52a or the shoulder may be formed so that an installation position of the support member 60 can be obtained, namely, an operation space can be obtained, wherein the skirt part 62 of the valve member 68 can be bent upward.

Moreover, a second engaging protrusion 52b may be further formed, which supports the edge of the back surface of the head part 54 irrespective of the formation of the first engaging protrusion 52a or the shoulder. The second engaging protrusion 52b may be used to prevent any retraction so that a state where an edge portion of the front side of the head part 54 pressurizes the inner surface of the first engaging protrusion 52a or the shoulder or the needle holder 50.

If the installation of the filter unit 80 with respect to the needle holder 50 is finished, the assembling can be finished in such a way to insert the neck part 82a of the cylinder 82 or a ringer connection tube 94 of a ringer assembly through the opening of the rear side of the needle holder 50.

The operational relationship of the filter unit in the thusly assembled state will be described as an example. The injection needle "N" is stuck in the opened ample (not illustrated) in a typical way, and the piston 84 is moved backward, by which the medicinal fluid in the ample can be sucked into the cylinder 82.

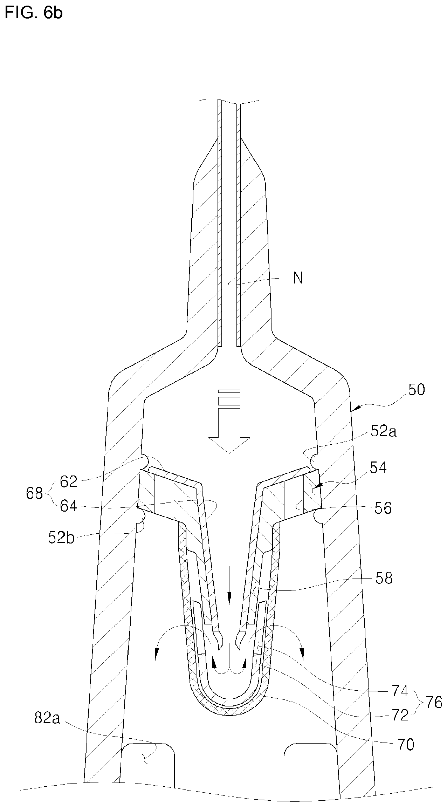

The flow of the medicinal fluid and impurities mixed through the injection needle "N", as illustrated in Figures 6A and 6B, may be concentrated on the central region "D1" which corresponds to the direction of the hollow part "M/H"' with the aid of a state wherein the through hole 56 is blocked by the skirt part 62 of the valve member 68 and the edge region "D2" which forms the shape sunk in the backward direction.

The cutting line 66 formed at the rear end of the extension part 64 of the valve member 68 can be split wide by means of the pressure, and the mixed medicinal fluid and impurities are passed.

The flow of the mixed medicinal fluid and impurities allow to push the cover member 76 in the backward direction, and the medicinal fluid and impurities pass through the filter 70 and between the cover legs 74 of the cover member 76, during which the impurities may be filtered by the filter 70 and may be collected at the inner side, and the passed medicinal fluid may be guided by the negative pressure which is continuously formed inside of the cylinder 82 and may enter the inside of the cylinder 82.

In a state where the medicinal fluid has been filled in the cylinder 82 through the aforementioned procedures, namely, if the negative pressure operation is finished due to the stop of the retraction of the piston 84, the cutting line 66 formed at the rear end of the extension part 64 of the valve member 68 may naturally return to the state where the cutting line 66 is closed through a shape recovery procedure.

The inside of the needle holder 50 is filled with the medicinal fluid, and the impurities filtered by the filter 70 is isolated between the side portion of the extension tube 58 including the rear end of the extension part 64 of the valve member 68 and the inner side of the filter 70, and the medicinal fluid accommodated in the cylinder 82 is a pure medicinal fluid from which the impurities have been removed.

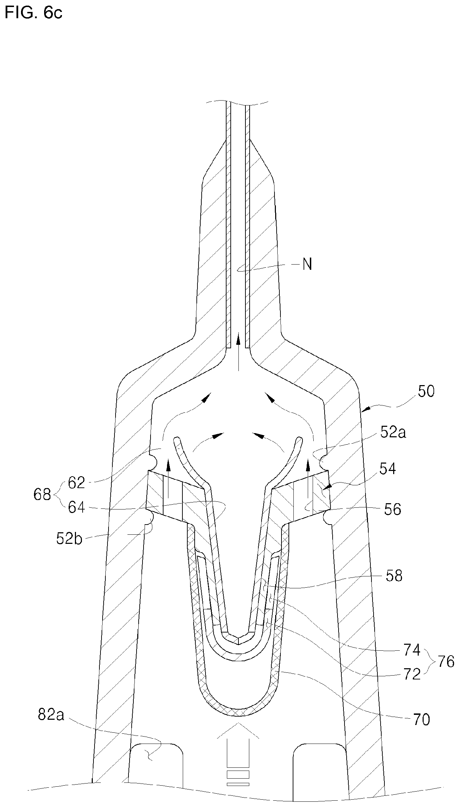

As illustrated in Figures 6A and 6C, during the administration procedure of the medicinal fluid based on the forward movement of the piston 84 with respect to the cylinder 82, the cover member 76 opposite to the neck part 82a of the cylinder 82 may move forward by receiving the flow pressure of the medicinal fluid based on the positive pressure inside the cylinder 82, and the cover part 72 of the cover member 76 may cover the rear end of the extension tube 58 including the rear end of the extension part 64 of the corresponding valve member 68, thus preventing the flow pressure of the medicinal fluid from affecting the rear end of the extension part 64.

The positive pressure starting from the inside of the cylinder 82 may deviate from the edge region "D2" due to the influence of the cover member 76 in the narrow central region "D1" where the rear end of the filter 70 70 positions, and the flow of the medicinal fluid passing through the edge region "D2"' may be concentrated in the direction of the through hole 56 since it receives the influence of the inclination formed by the inner surface of the needle holder 50 and the inclination formed by the inner surface of the extension tube 58.

Continuously, the flow pressure of the medicinal fluid passing through the through hole 56 may be administrated to a human body through the injection needle "N" while causing the skirt part 62 covering the circumferential portion of the front side of the through hole 56 to bend in the forward direction.

In the administration procedure of the medicinal fluid, the cover member 76 may allow to block the flow pressure of the medicinal fluid with respect to the rear end of the extension part 64 of the valve member 68, but it is not an essential component.

This configuration is related with a configuration wherein the inner area of the needle holder 50 expands backward, and since the rear end of the extension part 64 of the valve member 68 is formed becoming relatively narrower, the central region "D1"' occupied by the rear end of the extension part 64 of the valve member 68 is narrow as compared to the edge region "D2" of the outer side thereof, so the flow pressure caused due to the discharge of the medicinal fluid may be smaller as compared to the conventional technology.

Moreover, the central region "D1"' corresponding to the rear end of the extension part 64 of the valve member 68 may be first blocked by the filter 70 with respect to the flow pressure of the medicinal fluid, and since it is positioned in such a way that the inner side surface of the filter 70 covers the rear end of the extension part 64, the shape maintaining capability of the filter 70 can be further obtained in relation with the same.

Since the medicinal fluid to be administrated in this way is discharged after the impurities mixed with the medicinal fluid from the ample, etc. has been filtered and collected, the reliability on the blocking of the impurities can be enhanced.

Meanwhile, the syringe including the filter assembly for a syringe according to the present invention may include, but is not limited to, a cylinder 82 which forms a neck part 82a of the cylinder in a single tube type at the front end thereof and a space to accommodate a medicinal fluid, a piston 84 which is inserted from a rear opening of the cylinder 82 and is movable back and forth in the longitudinal direction and is able to provide a flow pressure of the medicinal fluid with respect to the inside of the cylinder 82 based on the movement thereof, and the filter assembly for a syringe formed of an engagement of the needle holder 50 and the filter unit 80.

Moreover, the ringer assembly including a filter assembly for a syringe according to the present invention may include, but is not limited to, a ringer body 90 configured to accommodate a ringer's solution, a ringer pipe 92 which forms a ringer's solution flow passage in the ringer body 90, a ringer connection tube 94 engaged to the ringer pipe 92, thus allowing the discharge of the ringer's solution, and the filter assembly for syringe formed of an engagement of the needle holder 50 and the filter unit 80.

## Claims

1. A filter assembly for a syringe, comprising:
a needle holder configured to fix an injection needle at a front end thereof, wherein a neck part of a cylinder is inserted into a rear end thereof; and
a filter unit which is disposed between the needle holder and the front end of the neck part, wherein the filter unit includes:
a support member which forms a hollow part formed passing through the center thereof in the forward and backward directions and is formed of a head part which contacts close with along the surrounding of an inner surface of the needle holder corresponding to a side surface which extends and protrudes outward from the front portion and is equipped with a through hole between the hollow part and an outer edge while passing through in the forward and backward directions, and an extension tube which extends backward toward the back surface of the head part;
a valve member which is formed in a horn shape having a hollow part, wherein a front end of the valve member forms a skirt part shaped to cover a circumferential portion of the through hole, and a rear end thereof which extends backward from the hollow circumferential portion of the back surface of the skirt part reaches the hollow rear end of the support member or is inserted in a protrusion shape and includes an extension part which forms a cutting line in the center of the rear end thereof; and
a filter wherein a front opening formed in a pocket shape is engaged in a shape covering the side wall of the extension tube.

2. The assembly of claim 1, wherein the inner wall of the needle holder includes at least a first engaging protrusion between the first engaging protrusion formed corresponding to an edge of the front surface of the head part at a previously set position and a second engaging protrusion corresponding to an edge of the back surface of the head part at a previously set position, and the skirt part is configured in such a way that the outer edge positions within an inner diameter defined by the first engaging protrusion in an installed state, and positions at an outer side of an outer line of the through hole.

3. The assembly of claim 1, wherein the needle holder forms a slope angle wherein the inner surface thereof is gradually expanding in the backward direction, and the head part contacts close with the inner surface of the needle holder corresponding at a slope angle wherein the outer side surface is gradually becoming narrow in the upward direction.

4. The assembly of claim 1, wherein the head part is formed in such a way that a front surface thereof is formed sunk in the backward direction gradually from an outer end to the hollow part, and the skirt part is configured in such a way that the thickness thereof is gradually decreasing in the direction from the hollow part to the outer side edge, and at least the back surface of the skirt part closely corresponds to the sunk shape of the front surface of the head part.

5. The assembly of claim 1, wherein the extension tube of the support member is configured in such a way that the inner and outer thicknesses thereof are gradually decreasing in the backward direction.

6. The assembly of claim 1, further comprising:
a cover member wherein a rear end thereof forms a dome-shaped cover part covering an outer side of the rear end of the extension tube including the rear end of the extension part, wherein the cover member includes two or more than two cover lags which are disposed at an interval along the surrounding of the front end of the cover part and are formed extending and protruding in the forward direction, and the cover member is configured to move to the rear end in the filter by the flow pressure of the medicinal fluid in the direction of the cylinder through the rear end of the valve member during the suction procedure and is able to guide the flow of the medicinal fluid between the cover legs and is configured to move forward in response to the flow of the medicinal fluid in the direction of an injection needle during an administration procedure, wherein the cover member is configured to guide the flow of the medicinal fluid in the direction of the through hole by covering the rear end of the extension tube.

7. A syringe, comprising:
a cylinder which forms a single tube-shaped cylinder neck part at a front end thereof and a space to accommodate a medicinal fluid;
a piston which is inserted through a rear opening of the cylinder and is able to move back and forth in the longitudinal direction, thus providing a flow pressure of the medicinal fluid to the inside of the cylinder based on the movement thereof;
a needle holder of claim 1; and
a filter unit of claim 1.

8. A ringer assembly, comprising:
a ringer body configured to accommodate a ringer's solution;
a ringer pipe which forms a flow passage of the ringer's solution in the ringer body;
a ringer connection tube which is engaged to the ringer pipe and is able to carry out the discharge of the ringer's solution;
a needle holder of claim 1; and
a filter unit of claim 1.
